# EUROPÄISCHE PATENTSCHRIFT

(11) **EP 0 263 464 B1**
(45) Veröffentlichungstag und Bekanntmachung des Hinweises auf die Patenterteilung: **14.05.1997**
(21) Anmeldenummer: 87114505.8
(22) Anmeldetag: 05.10.1987
(51) Int. Cl.: C07D 213/12, B01J 29/08

(54) **Verfahren zur Herstellung von substituierten Pyridinen**
Process for the preparation of substituted pyridines
Procédé pour la préparation de pyridines substituées

(30) Priorität: 08.10.1986 DE 3634259
(43) Veröffentlichungstag der Anmeldung: 13.04.1988
(73) Patentinhaber: DEGUSSA AG, 60311 Frankfurt (DE)
(72) Erfinder: Hölderich, Wolfgang, Dr., D-6710 Frankenthal (DE); Goetz, Norbert, Dr., D-6520 Worms 1 (DE); Fouquet, Gerd, Dr., D-6730 Neustadt (DE)

(56) Entgegenhaltungen:
- EP-A- 0 131 887
- EP-A- 0 232 182
- DE-A- 2 703 070

## Beschreibung

Die Erfindung betrifft ein Verfahren zur Herstellung von substituierten Pyridinen durch katalytische Umsetzung von Gemischen aus Acrolein und Alkanalen mit Ammoniak in Gegenwart von aciden Zeolithen des Pentasiltyps.

Es ist bekannt, daß bei der Umsetzung von Acrolein mit Ammoniak in der Gasphase in Gegenwart von Katalysatoren 3-Methylpyridin entsteht. Als Katalysatoren dienen mit Sauerstoff bei Temperaturen von 550 bis 1200°C vorbehandelte Verbindungen aus den Elementen Al, F und O die zusätzlich mindestens ein Element der zweiten, dritten oder vierten Gruppe des Periodensystems (DE-OS 2 151 417) oder mindestens zwei Elemente der zweiten, vierten, fünften oder sechsten Gruppe des Periodensystems (DE-OS 2 224 160) oder mindestens ein Element der zweiten Hauptgruppe des Periodensystems (DE-OS 2 239 801) enthalten. Es ist auch bekannt, bei Ausführung der Umsetzung im Wirbelbett das Acrolein getrennt vom Ammoniak in die Wirbelschicht einzuspeisen. Nachteilig ist bei diesen Verfahren, daß neben 3-Methylpyridin in erheblichem Umfang Pyridin entsteht.

In bescheidenen Ausbeuten erhält man 3-Methylpyridin auch durch Umsetzung von Gemischen von Acrolein und Propionaldehyd mit Ammoniak in Gegenwart von Katalysatoren, die Aluminiumoxide und Siliciumoxide enthalten. Durch Einsatz hochdisperser Aluminiumsilikate kann die Ausbeute an 3-Methylpyridin etwas gesteigert werden (DE-OS 27 03 070).

In US 4 220 783 wird ein Verfahren zur Herstellung von Pyridin und Picolin an Aluminosilikatzeolith ZSM 5 durch Umsetzung von C₂-C₄-Aldehyden oder C₃-C₅-Ketonen mit Ammoniak in Gegenwart von Methanol oder Wasser beschrieben. Der Katalysator wird sehr schnell desaktiviert. Die Ausbeuten sind unbefriedigend.

Aus EP 131 887 ist bekannt, daß acide Aluminosilikatzeolithe vom Pentasiltyp mit einem Constraint Index von 1 bis 12 im Wirbelbett bei der Herstellung von Alkylpyridinen bessere Ergebnisse liefern als im Festbett. Bei der Umsetzung von Acetaldehyd mit Formaldehyd erreicht die Gesamtausbeute der erhaltenen Pyridine 89,8 %, wobei das Pyridin/β-Picolin-Verhältnis gleich 2:1 ist. Die Reaktionstemperatur liegt über 450°C und der Katalysator muß bereits nach 4 Std. regeneriert werden.

Die EP-A - 0 232 182 betrifft ein Verfahren insbesondere von Pyridin, das nur dann in hohen Ausbeuten erhalten wird, wenn der als Katalysator verwendete Pentasilzeolith mit Tl, Pb oder Co dotiert wurde.

Es war daher die Aufgabe gestellt, substituierte Pyridine aus gut zugänglichen Ausgangsstoffen selektiv, ohne einen größeren Zwangsanfall an Pyridin zu synthetisieren.

Es wurde nun gefunden, daß man substituierte Pyridine der Formel (I) 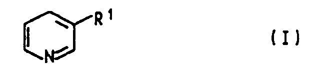 in der R¹ Alkyl- mit 1 bis 20 Kohlenstoffatomen, Cycloalkyl-. Aryl- oder Aralkylrest bedeutet, durch Umsetzung eines Gemisches von Acrolein und Alkanalen der Formel (II) 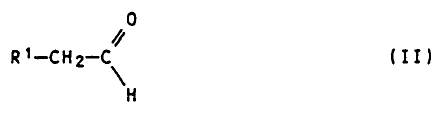 in der R¹ obige Bedeutung hat, mit Ammoniak in einfacher Weise erhält, wenn man die Reaktion in Gegenwart von aciden Zeolithen des Pentasiltyps als Katalysatoren durchführt.

Die Reaktion wird in der Gasphase bei einer Temperatur von 150 bis 400°C ausgeführt. Erfindungsgemäß wird ein Gemisch von Acrolein und Alkanalen der Formel (II) umgesetzt. Geeignete Alkanale sind z.B. Propionaldehyd, Butyraldehyd, Isobutyraldehyd, Pentanal, Hexanal, Octanal, Phenylacetaldehyd, 3-Phenylpropanal, Cyclohexylacetaldehyd und Cyclopentylacetaldehyd.

Die erfindungsgemäße Umsetzung läßt sich z.B. für den Fall der Herstellung von 3-Ethylpyridin durch folgende Formelgleichung wiedergeben:

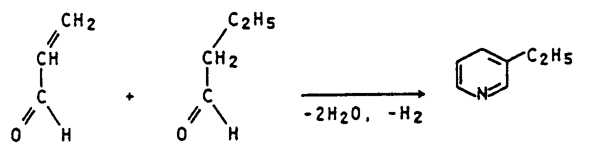

Die Durchführung der erfindungsgemäßen Reaktion in Gegenwart von Zeolithen, die normalerweise Crackeigenschaften haben, ist insofern überraschend, als man bei der Umsetzung längerkettiger Alkanale verstärkt mit Kettenabbruch rechnen müßte.

Als Katalysatoren für das erfindungsgemäße Verfahren verwendet man die Zeolithe zweckmäßig in der aciden Form. Zeolithe sind kristalline Aluminiumsilikate, die eine hochgeordnete Struktur mit einem starren dreidimensionalen Netzwerk von SiO₄- und AlO₄-Tetraedern besitzen, die durch gemeinsame Sauerstoffatome verbunden sind. Das Verhältnis der Si-und Al-Atome zu Sauerstoff beträgt 1 : 2. Die Elektrovalenz der Aluminium enthaltenden Tetraeder ist durch Einschluß von Kationen in den Kristall. z.B. eines Alkali- oder Wasserstoffions ausgeglichen. Ein Kationenaustausch ist möglich. Die Räume zwischen den Tetraedern sind vor der Dehydration durch Trocknen bzw. Calcinieren von Wassermolekülen besetzt.

In den Zeolithen können anstelle von Aluminium auch andere Elemente wie B, Ga, Fe, Cr, V, As, Sb, Bi oder Be oder deren Gemische in das Gitter eingebaut werden, oder das Silicium kann durch ein vierwertiges Element wie Ge, Ti, Zr, Hf ersetzt werden.

Entsprechend ihrer Struktur werden Zeolithe in verschiedene Gruppen unterteilt. So bilden bei der Mordenit-Gruppe Ketten oder bei der Chabasit-Gruppe Schichten aus Tetraedern die Zeolith-Struktur. während sich bei der Faujasit-Gruppe die Tetraeder zu Polyedern ordnen. z.B. in Form eines Kubooktaeders, der aus Vierringen bzw. Sechsringen aufgebaut ist. Je nach Verknüpfung der Kubooktaeder, wodurch unterschiedlich große Hohlräume und Poren entstehen, unterscheidet man in Zeolithe vom Typ A, L, X oder Y.

Für das erfindungsgemäße Verfahren in Betracht kommende Katalysatoren sind Zeolithe aus der Mordenit-Gruppe oder engporige Zeolithe vom Erionit-bzw. Chabasit-Typ oder Zeolithe vom Faujasit-Typ. z.B. Y-, X- oder L-Zeolithe. In dieser Gruppe von Zeolithen gehören auch die sogenannten "ultrastabilen" Zeolithe des Faujasittyps, d.h. dealuminierte Zeolithe. Verfahren zur Herstellung solcher Zeolithe sind mannigfach beschrieben.

Geeignet sind acide Zeolithe vom Pentasiltyp. Diese haben als Grundbaustein einen aus SiO₄-Tetraedern aufgebauten Fünfring gemeinsam. Sie sind durch eine hohes SiO₂/Al₂O₃-Verhältnis gekennzeichnet sowie durch Porengrößen. die zwischen denen der Zeolithe vom Typ A und denen vom Typ X oder Y liegen.

Die Zeolithe vom Pentasiltyp können unterschiedliche chemische Zusammensetzung aufweisen. Es handelt sich hierbei um Alumino-, Boro-, Eisen-, silikatzeolithe oder deren Gemische.

Dabei sind die aciden Aluminiumsilikatzeolithe mit einer Säure und/oder Wasserdampf nachbehandelt oder mit Übergangsmetallen oder Seltenen Erden dotiert.

Der Aluminosilikatzeolith wird z.B. aus einer Aluminiumverbindung. vorzugsweise Al(OH)₃ oder Al₂(SO₄)₃ und einer Siliciumkomponente, vorzugsweise hochdispersem Siliciumdioxid in wäßriger Aminlösung, insbesondere in Polyaminen wie 1,6-Hexandiamin- oder 1,3-Propandiamin-oder Triethylentetramin-Lösung mit oder insbesondere ohne Alkali- oder Erdalkalizusatz bei 100 bis 220°C unter autogenem Druck hergestellt. Hierzu gehören auch die isotaktischen Zeolithe nach EP 34 727 und EP 46 504. Die erhaltenen Aluminosilikatzeolithe enthalten je nach Wahl der Einsatzstoffmengen ein SiO₂/Al₂O₃-Verhältnis von 10 bis 40 000. Diese Aluminosilikatzeolithe des Pentasiltyps können auch in etherischem Medium wie Diethylenglykoldimethylether, in alkoholischem Medium wie Methanol bzw. 1,4-Butandiol oder in Wasser synthetisiert werden.

Zu den erfindungsgemäß verwendbaren siliciumreichen Zeolithen (SiO₂/Al₂O₃ ≥ 10) gehören auch die verschiedenen ZSM-Typen, Ferrierit, Nu-1 und Silicalit ®, unter den oben genannten Voraussetzungen.

Borosilikatzeolithe kann man z.B. bei 90 bis 200°C unter autogenem Druck synthetisieren, indem man eine Borverbindung, z.B. H₃BO₃, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit und insbesondere ohne Alkali- oder Erdalkalizusatz zur Reaktion bringt. Hierzu gehören auch die isotaktischen Zeolithe nach EP 34 727 und EP 46 504. Solche Borosilikatzeolithe können ebenfalls hergestellt werden, wenn man die Reaktion statt in wäßriger Aminlösung in etherischer Lösung. z.B. Diethylenglykoldimethylether oder in alkoholischer Lösung. z.B. 1,6-Hexandiol durchführt.

Den Eisensilikatzeolith erhält man z.B. aus einer Eisenverbindung. vorzugsweise Fe₂(SO₄)₃ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 200°C unter autogenem Druck.

Die so hergestellten Alumino-. Boro- und Eisensilikatzeolithe können nach ihrer Isolierung. Trocknung bei 100 bis 160°C. vorzugsweise 110°C und Calcinierung bei 450 bis 550°C. vorzugsweise 500°C. mit einem Bindemittel im Verhältnis 90 : 10 bis 40 : 60 Gew.-% zu Strängen oder Tabletten verformt werden. Als Bindemittel eignen sich diverse Aluminiumoxide, bevorzugt Boehmit, amorphe Aluminosilikate mit einem SiO₂/Al₂O₃-Verhältnis von 25 : 75 bis 90 : 5, bevorzugt 75 : 25, Siliciumdioxid, bevorzugt hochdisperses SiO₂, Gemische aus hochdispersem SiO₂ und hochdispersem Al₂O₃, TiO₂, ZrO₂ sowie Ton. Nach der Verformung werden die Extrudate oder Preßlinge bei 110°C/16 h getrocknet und bei 500°C/16 h calciniert.

Man erhält auch vorteilhafte Katalysatoren, wenn der isolierte Alumino- bzw., Borosilikatzeolith direkt nach der Trocknung verformt wird und erst nach der Verformung einer Calcinierung unterworfen wird. Die hergestellten Alumino- und Borosilikatzeolithe können in reiner Form, ohne Binder, als Stränge oder Tabletten eingesetzt werden, wobei als Verstrangungs- oder Peptisierungshilfsmittel, z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden.

Liegt der Zeolith aufgrund der Art seiner Herstellung nicht in der katalytisch aktiven, aciden H-Form vor, sondern z.B. in der Na-Form, dann kann diese durch Ionenaustausch, z.B. mit Ammoniumionen und anschließende Calcinierung oder durch Behandlung mit Säuren vollkommen oder partiell in die gewünschte H-Form überführt werden.

Wenn bei der erfindungsgemäßen Verwendung der zeolithischen Katalysatoren eventuell eine durch Koksabscheidung bedingte Desaktivierung eintritt, empfiehlt es sich, die Zeolithe durch Abbrennen der Koksablagerung mit Luft oder mit einem Luft/N₂-Gemisch bei 400 bis 550°C, bevorzugt 500°C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Um eine möglichst hohe Selektivität, hohen Umsätzen sowie lange Standzeiten zu erreichen, ist es vorteilhaft, die Zeolithe zu modifizieren. Eine geeignete Modifizierung der Katalysatoren besteht z.B. darin, daß man den unverformten oder verformten Zeolithen mit Metallsalzen durch einen Ionenaustausch oder durch Imprägnierung dotiert. Als Metalle werden Alkalimetalle wie Li, Cs, K, Erdalkalimetall wie Mg, Ca, Sr, Metalle der 3., 4. und 5. Hauptgruppe wie Al, Ga, Ge, Sn, Pb, Bi, Übergangsmetalle der 4. - 8. Nebengruppe wie Ti, Zr, V, Nb, Cr, Mo, W, Mn, Re, Fe, Ru, Os, Co, Rh, Sr, Ni, Pd, Pt, Übergangsmetalle der 1. und 2. Nebengruppe wie Cu, Ag, Zn, Seltene Erdmetalle wie La, Ce, Pr, Nd, Er, Yb und U eingesetzt.

Zweckmäßigerweise führt man die Dotierung so durch, daß man den verformtan Zeolithen in einem Steigrohr vorlegt und bei 20 bis 100°C eine wäßrige oder ammonialkalische Lösung eines Halogenids oder eines Nitrats der voranbeschriebenen Metalle überleitet. Ein derartiger Ionenaustausch kann an der Wasserstoff-, Ammonium- und Alkaliform des Zeolithen vorgenommen werden. Eine weitere Möglichkeit der Metallaufbringung auf den Zeolithen ist gegeben, indem man das zeolithische Material, z.B. mit einem Halogenid, einem Nitrat oder einem Oxid der voranbeschriebenen Metalle in wäßriger, alkoholischer oder ammonialkalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht z.B. darin, daß man Cu(NO₃)₂ x 3 H₂O oder Ni(NO₃)₂ x 6 H₂O oder Ce(NO₃)₃ x 6 H₂O oder La(NO₃)₂ x 6 H₂O oder Cs₂CO₃ in Wasser löst und mit dieser Lösung den verformten oder unverformten Zeollith eine gewisse Zeit, z.B. 30 Minuten, tränkt. Die eventuell überstehende Lösung wird im Rotationsverdampfer von Wasser befreit. Danach wir der getränkte Zeolith bei etwa 150°C getrocknet und bei etwa 550°C calciniert. Dieser Tränkvorgang kann mehrmals hintereinander vorgenommen werden, um den gewünschten Metallgehalt einzustellen.

Es ist auch möglich, eine wäßrige Ni(NO₃)₂-Lösung oder ammoniakalische Pd(NO₃)₂-Lösung herzustellen und darin den reinen pulverförmigen Zeolithen bei 40 bis 100°C unter Rühren etwa 24 h aufzuschlämmen. Nach Abfiltrieren, Trocknen bei etwa 150°C und Calcinierung bei etwa 500°C kann das so gewonnene zeolithische Material mit oder ohne Bindemittel zu Strängen, Pellets oder Wirbelgut weiterverarbeitet werden.

Ein Ionenaustausch des in der H-Form oder Ammonium-Form oder Alkali-Form vorliegenden Zeolithen kann so vorgenommen werden, daß man den Zeolithen in Strängen oder Pellets in einer Kolonne vorlegt und darüber z.B. eine wäßrige Ni(NO₃)₂-Lösung oder ammoniakalische Pd(NO₃)₂-Lösung bei leicht erhöhter Temperatur zwischen 30 und 80°C im Kreislauf 15 bis 20 h leitet. Danach wird mit Wasser ausgewaschen, bei etwa 150°C getrocknet und bei etwa 550°C calciniert. Bei manchen metalldotierten Zeolithen, z.B. Pd-, Cu-, Ni-dotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft. Dabei geht man vorteilhaft, z.B. so vor, daß man Zeolithe in Pulverform mit 1 n Phosphorsäure 1 Stunde bei 80°C behandelt. Nach der Behandlung wird mit Wasser gewaschen, bei 110°C/16 Stunden getrocknet und bei 500°C/20 Stunden calciniert. Nach einer anderen Arbeitsweise behandelt man Zeolithe vor oder nach ihrer Verformung mit Bindemittel, z.B. 1 bis 3 Stunden bei Temperaturen von 60 bis 80°C mit einer 3 bis 25 Gew.-%igen, insbesondere 12 bis 20 Gew.-%igen wäßrigen Salzsäure. Anschließend wird der so behandelte Zeolith mit Wasser gewaschen, getrocknet und bei 400°C bis 500°C calciniert.

Eine besondere Ausführungsform für die Säurebehandlung besteht darin, daß man das zeolithische Material vor seiner Vorformung bei erhöhter Temperatur mit Flußsäure, die im allgemeinen als 0,001 n bis 2 n, vorzugsweise 0,05 n bis 0,5 n Flußsäure eingesetzt wird, behandelt, beispielsweise durch Erhitzen unter Rückfluß über einen Zeitraum von im allgemeinen 0,5 bis 5, vorzugsweise 1 bis 3 Stunden. Nach Isolierung, z.B. durch Abfiltrieren und Auswaschen des zeolithischen Materials, wird dieses zweckmäßig, z.B. bei Temperaturen von 100 bis 160°C, getrocknet und bei Temperaturen von im allgemeinen 450°C bis 600°C calciniert. Gemäß einer weiteren bevorzugten Ausführungsform für die Säurebehandlung, wird das zeolithische Material nach einer Verformung mit Bindemittel bei erhöhter Temperatur, zweckmäßig bei Temperaturen von 50 bis 90°C, vorzugsweise 60 bis 80°C, über einen Zeitraum von 0,5 bis 5 h, vorzugsweise mit 12 bis 20 Gew.-%iger Salzsäure, behandelt. Zweckmäßig wird das zeolithische Material anschließend ausgewaschen, bei Temperaturen von 100 bis 160°C getrocknet und bei Temperaturen von 450 bis 600°C calciniert. Einer HF-Behandlung kann sich auch eine HCl-Behandlung anschließen.

Die hier beschriebenen Katalysatoren können wahlweise als 2- bis 4-mm-Stränge oder als Tabletten mit 3 bis 5 mm Durchmesser oder als Splitt mit Teilchengrößen von 0,1 bis 0,5 mm oder als Wirbelkontakt eingesetzt werden.

Für die Umsetzung wird zweckmäßig ein Molverhältnis von Acrolein zu Alkanal zu NH₃ von 1:1-5:1-10, insbesondere 1:1-2:1-5 eingestellt. Im allgemeinen arbeitet man in der Gasphase bei Temperaturen von 100 bis 500°C, vorteilhaft von 150 bis 450°C, insbesondere bei 200 bis 400°C und bei einem Druck von 0,1 bis 100 bar, insbesondere 0,5 bis 10 bar. In der Gasphase wird vorteilhaft eine Katalysatorbelastung von 0,1 bis 20, insbesondere von 1 bis 90 g Gemisch Acrolein und Alkanal je g Katalysator und Stunde ein (WHSV = g Einsatzgemisch/g Katalysator und Stunde) eingestellt. Die Gasphasenreaktion kann in einem Festbett oder in einem Wirbelbett ausgeführt werden. Es ist auch möglich, die Reaktion in der Flüssigphase (Suspension-, Riesel- oder Sumpffahrweise) bei Temperaturen zwischen 50 und 200°C durchzuführen. Die Umsetzung kann kontinuierlich und diskontinuierlich erfolgen.

Schwerflüchtige oder feste Ausgangsstoffe werden zweckmäßig in gelöster Form z.B. in THF-, Toluol- oder Petrolether-Lösung eingesetzt. Allgemein ist eine Verdünnung des Ausgangsstoffes mit Lösungsmitteln oder mit Inertgasen wie N₂, Ar, H₂O-Dampf möglich. Es empfiehlt sich, bei dieser Reaktion auch in Gegenwart von Sauerstoff zu arbeiten.

Die Endprodukte werden durch übliche Verfahren, z.B. durch Destillation aus dem Reaktionsgemisch isoliert; nichtumgesetztes Einsatzgemisch wird gegebenenfalls in Umsetzung zurückgeführt.

Die nach dem erfindungsgemäßen Verfahren erhältlichen substituierten Pyridine sind vielseitig verwendbare Zwischenprodukte, z.B. für Farbstoffe, Pharmazeutika, Schädlingsbekämpfungsmittel und wertvolle Lösungsmittel.

### Beispiele 1 bis 40

Die Reaktion wird in der Gasphase unter isothermen Bedingungen in einem Rohrreaktor (Wendel, 0,6 cm Innendurchmesser, 90 cm Länge) mindestens 6 Stunden lang durchgeführt. Die Reaktionsprodukte werden durch übliche Methoden abgetrennt und charakterisiert. Die quantitative Bestimmung der Reaktionsprodukte und der Ausgangsprodukte erfolgt gaschromatographisch nach bekannter Methode.

In den Beispielen werden folgende Katalysatoren eingesetzt:

### Katalysator A

Ein Borosilikatzeolith des Pentasil-Typs wird in einer hydrothermalen Synthese aus 640 g hochdispersem SiO₂, 122 g H₃BO₃, 8000 g einer wäßrigen 1,6-Hexandiamin-Lösung (Mischung 50:50 Gew.%) bei 170°C unter autogenem Druck in einem Rührautoklaven hergestellt. Nach Abfiltrieren und Auswaschen wird das kristalline Reaktionsprodukt bei 100°C/24 h getrocknet und bei 500°C/24 h calciniert. Dieser Borosilikatzeolith setzt sich zusammen aus 94,2 Gew.% SiO₂ und 2,3 Gew.% B₂O₃.

Mit diesem Material werden durch Verformen mit Boehmit im Gewichtsverhältnis 60:40 2 mm-Stränge hergestellt, die bei 110°C/16 h getrocknet und bei 500°C/24 h calciniert werden.

### Katalysator B

Ein Aluminosilikatzeolith vom Pentasil-Typ wird unter hydrothermalen Bedingungen bei autogenem Druck und 150°C aus 65 g hochdispersem SiO₂, 20,3 g Al₂(SO₄)₃ x 18 H₂O in 1 kg einer wäßrigen 1,6-Hexandiamin-Lösung (Mischung 50:50 Gew.%) in einem Rührautoklaven hergestellt. Nach Abfiltrieren und Auswaschen wird das kristalline Reaktionsprodukt bei 110°C/24 h getrocknet und bei 500°C/24 h calciniert. Dieser Aluminosilikatzeolith enthält 91,6 Gew.% SiO₂ und 4,6 Gew.% Al₂O. Der Katalysator wird durch Verformen mit Boehmit im Gewichtsverhältnis 60:40 zu 2 mm-Strängen erhalten, bei 110°C/16 h getrocknet und bei 500°C/24 h calciniert.

### Katalysator C

Katalysator B wird mit einer wäßrigen La(NO₃)₃-Lösung imprägniert, bei 130°C/2 h trocknet und bei 540°C/2 h calciniert. Der La-Gehalt beträgt 3,2 Gew.%.

### Katalysator D

Katalysator B wird mit einer wäßrigen La(NO₃)₃-Lösung imprägniert, bei 130°C/2 h getrocknet und bei 540°C/2 h calciniert. La-Gehalt beträgt 3,25 Gew.%.

### Katalysator E

100 g des für Katalysator A verwendeten Borosilikatzeolithen werden mit 280 ml einer 0,1 n HF bei 90°C 2 h lang behandelt und nach Abfiltrieren bei 160°C getrocknet. Dieses Produkt wird mit amorphem Aluminosilikat (25 Gew.% Al₂O₃ und 75 Gew.% SiO₂) im Gewichtsverhältnis 60:40 zu 2 mm-Strängen verformt, bei 110°C/16 h getocknet und bei 500°C/16 h calciniert.

### Katalysator F

Katalysator A wird mit wäßriger Co(NO₃)₂-Lösung imprägniert, bei 130°C/2 h getrocknet und bei 540°C/2 h calciniert. Der Co-Gehalt beträgt 3,2 Gew.%.

### Katalysator G

Katalysator A wird mit wäßriger Ce(NO₃)₃-Lösung imprägniert, bei 130°C/2 h getrocknet und bei 540°C/2 h calciniert. Der Ce-Gehalt beträgt 2,5 Gew.%.

### Katalysator H

Katalysator H wird erhalten, indem man den Aluminosilikatzeolith des Katalysators B mit einem Verformungshilfsmittel verformt, bei 110°C/16 h trocknet und bei 500°C/24 h calciniert und danach mit wäßriger Ce(NO₃)₃-Lösung imprägniert, bei 130°C/2 h trocknet und bei 540°C/2 h calciniert. Der Ce-Gehalt beträgt 3,4 Gew.%.

### Katalysator I

49 Gew.% Baylith® (® eingetragenes Warenzeichen) werden mit 21 Gew.% amorphem Aluiniumsilikat (45 Gew.% Al₂O₃ und 55 Gew.% SiO₂), 20 Gew.% MoO₃ und 10 Gew.% NiO zu Strängen verformt.

### Katalysator J

Handelsüblicher NaY-Zeolith wird mit Boehmit im Gewichtsverhältnis 60:40 zu 2 mm-Strängen verformt, bei 110°C/16 h getrocknet und bei 540°C/24 h calciniert. Diese Stränge werden mit einer 20 %igen wäßrigen La(NO₃)₂-Lösung bei 80°C/2 h ionenausgetauscht. Nach Trocknung bei 100°C und Calcination bei 500°C soll der La-Gehalt 7,1 Gew.% und der Na-Gehalt 1,1 Gew.% betragen. Der Ionenaustausch kann nach Zwischencalcination wiederholt werden bis obige La- bzw. Na-Gehalt eingestellt sind.

### Katalysator K

Wie bei Katalysator B beschrieben, wird ein Aluminosilikatzeolith hergestellt, jedoch wird statt 1,6-Hexandiamin eine wäßrige 1,3-Diaminopropan-Lösung verwendet. Das bei 140°C getrocknete und bei 500°C/24 h calcinierte Pulver enthält 89,9 Gew.% SiO₂ und 4,0 Gew.% Al₂O₃. Dieses Pulver wird mit 0,1 n HF 1 h unter Rückfluß behandelt. Nach Auswaschen mit H₂O, Trocknung bei 110°C und Calcination bei 500°C/5 h wird der so behandelte Aluminosilikatzeolith mit amorphem Aluminosilikat (75 Gew.% SiO₂, 25 Gew.% Al₂O₃) im Gewichtsverhältnis 60:40 zu 3 mm Strängen verformt, bei 110°C getrocknet und bei 500°C/16 h calciniert.

### Katalysator L

Katalysator L wird hergestellt wie Katalysator M, jedoch wird als Binder Boehmit statt amorphes Aluminosilikat verwendet.

### Katalysator M

Katalysator B wird mit einer wäßrigen RhCl₃-Lösung imprägniert, bei 130°C/2 h getrocknet und bei 540°C/2 h calciniert. Der Rh-Gehalt beträgt 2,84 Gew.%.

### Katalysator N

Katalysator A wird mit einer wäßrigen Co(NO₃)₂-Lösung und einer wäßrigen Ca(NO₃)₂-Lösung imprägniert, bei 130°C/2 h getrocknet und bei 540°C/2 h calciniert. Der Co-Gehalt beträgt 1,1 Gew.%, der Ca-Gehalt 0,1 Gew.%.

Die Versuchsergebnisse und die Reaktionsbedingungen sind in den folgenden Tabellen zusammengestellt.

**Tabelle 1**

| Acrolein : n-Butanal : NH₃ = 1 : 1 : 3 molar | | | | | | | | | | |
|---|---|---|---|---|---|---|---|---|---|---|
| Beispiel | 1 | 2 | 3 | 4 | 5 | 6 | 7 | 8 | 9 | 10 |
| Katalysator | A | B | B | B | C | E | E | D | I | J |
| Temperatur | 400°C | 350°C | 400°C | 400°C | 400°C | 350°C | 400°C | 400°C | 400°C | 400°C |
| WHSV | 3 h-¹ | 3 h-¹ | 3 h-¹ | 1,5 h-¹ | 3 h-¹ | 3 h-¹ | 3 h-¹ | 3 h-¹ | 3 h-¹ | 3 h-¹ |
| Umsatz % ¹⁾ | 100 | 100 | 100 | 100 | 100 | 100 | 100 | 100 | 100 | 100 |

| Selektivität % | | | | | | | | | | |
|---|---|---|---|---|---|---|---|---|---|---|
| 3-Ehylpyridin | 70,0 | 68,6 | 65,7 | 67,7 | 67,6 | 67,2 | 72,0 | 67,9 | 68,0 | 71,4 |
| β-Picolin | 15,6 | 15,1 | 14,4 | 16,5 | 16,6 | 15,1 | 15,4 | 16,2 | 10,7 | 16,4 |

| | | | | | | | | | | |
|---|---|---|---|---|---|---|---|---|---|---|
| 1) Umsatz sowohl von Acrolein als auch n-Butanal | | | | | | | | | | |

**Tabelle 2**

| Acrolein : n-Hexanal : NH₃ = 1 : 1 : 3 molar | | | | | |
|---|---|---|---|---|---|
| Beispiel | 11 | 12 | 13 | 14 | 15 |
| Katalysator | B | B | E | E | J |
| Temperatur | 350°C | 400°C | 350°C | 350°C | 400°C |
| WHSV | 3 h-¹ | 3 h-¹ | 3 h-¹ | 6 h-¹ | 3 h-¹ |
| Umsatz % ¹⁾ | 100 | 100 | 100 | 100 | 100 |

| Selektivität % | | | | | |
|---|---|---|---|---|---|
| 3-Butylpyridin | 76,8 | 69,0 | 76,2 | 77,6 | 74,1 |
| β-Picolin | 12,2 | 14,7 | 9,7 | 12,8 | 14,0 |

| | | | | | |
|---|---|---|---|---|---|
| 1) Umsatz sowohl von Acrolein als auch n-Hexanal | | | | | |

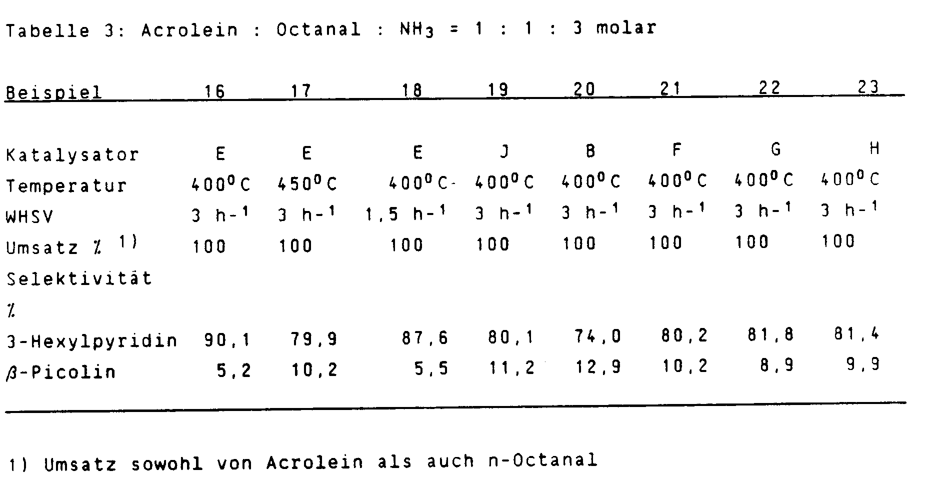

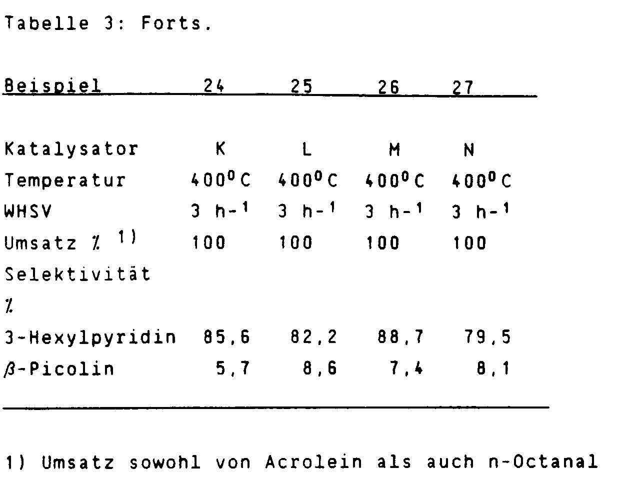

**Tabelle 4**

| Acrolein : Phenylacetaldehyd : NH₃ = 1 : 1 : 3 molar ¹⁾ | | | | | |
|---|---|---|---|---|---|
| Beispiel | 28 | 29 | 30 | 31 | 32 |
| Katalysator | A | D | B | E | J |
| Temperatur | 300°C | 300°C | 300°C | 300°C | 300°C |
| WHSV | 3 h-¹ | 3 h-¹ | 3 h-¹ | 3 h-¹ | 3 h-¹ |
| Umsatz % ²⁾ | 100 | 100 | 100 | 100 | 100 |

| Selektivität % | | | | | |
|---|---|---|---|---|---|
| 3-Phenylpyridin | 88,9 | 87,1 | 84,0 | 86,0 | 86,6 |

| | | | | | |
|---|---|---|---|---|---|
| ¹⁾ Gemisch aus Acrolein und Phenylacetaldehyd wird in THF gelöst 50 g : 50 9 | | | | | |
| ²⁾ Umsatz sowohl von Acrolein als auch Phenylacetaldehyd | | | | | |

**Tabelle 5**

| Acrolein : 3-Phenylpropanal : NH₃ = 1 : 1 : 3 molar ¹⁾ | | | | | |
|---|---|---|---|---|---|
| Beispiel | 33 | 34 | 35 | 36 | 37 |
| Katalysator | A | B | D | E | J |
| Temperatur | 300°C | 300°C | 300°C | 300°C | 300°C |
| WHSV | 3 h-¹ | 3 h-¹ | 3 h-¹ | 3 h-¹ | 3 h-¹ |
| Umsatz % ²⁾ | 100 | 100 | 100 | 100 | 100 |

| Selektivität % | | | | | |
|---|---|---|---|---|---|
| 3-Benzylpyridin | 86,3 | 83,1 | 84,7 | 89,7 | 90,2 |

| | | | | | |
|---|---|---|---|---|---|
| ¹⁾ Gemisch aus Acrolein und 3-Phenylpropanal wird in THF gelöst 50 g:50 g | | | | | |
| ²⁾ Umsatz sowohl von Acrolein als auch 3-Phenylpropanal | | | | | |

**Tabelle 6**

| Acrolein : n-Propanal : NH₃ = 1 : 1 : 3 molar | | | |
|---|---|---|---|
| Beispiel | 38 | 39 | 40 |
| Katalysator | A | B | E |
| Temperatur | 400°C | 400°C | 400°C |
| WHSV | 3 h-¹ | 3 h-¹ | 3 h-¹ |
| Umsatz % ¹⁾ | 100 | 100 | 100 |

| Selektivität % | | | |
|---|---|---|---|
| β-Picolin | 87,2 | 89,3 | 91,0 |

| | | | |
|---|---|---|---|
| ¹⁾ Umsatz sowohl von Acrolein als auch n-Propanal | | | |

### Beispiel 41

In einem Langzeitversuch wurde ein Gemisch aus Acrolein und n-Octanal mit Ammoniak im Molverhältnis 1:1:3 im voran beschriebenen Reaktor bei 400°C und WHSV = 3 h-¹ an Katalysator E umgesetzt. Während 48 h werden Acrolein und n-Octanal vollständig umgesetzt. Ein Aktivitätsverlust wurde nicht festgestellt. Die Selektivität an Hexylpyridin und β-Picolin liegt im Durchschnitt bei 93 %.

## Patentansprüche

1. Verfahren zur Herstellung von substituierten Pyridinen der Formel (I) 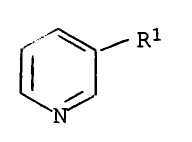 in der R¹ Alkyl mit 1 bis 20 Kohlenstoffatomen, Cycloalkyl, Aryl- oder Aralkylrest bedeutet, durch Umsetzung eines Gemisches von Acrolein und Alkanalen der Formel (II) 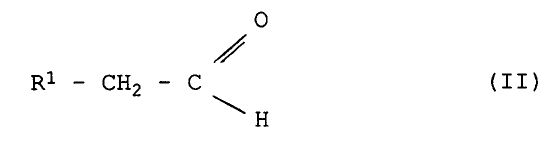 in der R¹ obige Bedeutung hat, mit Ammoniak in der Gasphase bei Temperaturen von 150 bis 400°C in Gegenwart von aciden Zeolithen des Pentasiltyps,
**dadurch gekennzeichnet**,
daß man Borsilikatzeolithe, Eisensilikatzeolithe und Aluminiumsilikatzeolithe des Pentasiltyps mit Ausnahme der mit Tl, Pb oder Co dotierten Typen verwendet, wobei die aciden Aluminiumsilikatzeolithe mit einer Säure und/oder Wasserdampf nachbehandelt oder mit Übergangsmetallen oder Seltenen Erden dotiert sind.

2. Verfahren gemäß Anspruch 1,
**dadurch gekennzeichnet**,
daß man mit Übergangsmetallen oder Seltenen Erden dotierte Bor- oder Eisensilikatzeolithe verwendet.

3. Verfahren nach Anspruch 1,
**dadurch gekennzeichnet**,
daß man mit einer Säure und/oder Wasserdampf nachbehandelte acide Bor- oder Eisensilikatzeolithe verwendet.

4. Verfahren nach einem oder mehreren der Ansprüche 1 bis 3,
**dadurch gekennzeichnet**,
daß man die Reaktion in einem Festbett durchführt.

5. Verfahren nach einem oder mehreren der Ansprüche 1 bis 3,
**dadurch gekennzeichnet**,
daß man die Reaktion in einem Wirbelbett durchführt.

## Claims

1. Process for the preparation of substituted pyridines corresponding to formula (I) 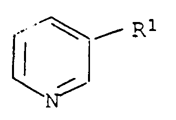 wherein R¹ denotes alkyl having 1 to 20 carbon atoms, cycloalkyl, aryl or aralkyl group, by reaction of a mixture of acrolein and alkanals corresponding to formula (II) 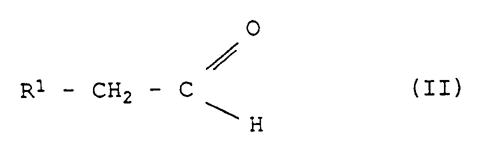 wherein R¹ has the above meaning, with ammonia in the gas phase at temperatures of from 150°C to 400°C in the presence of acidic zeolites of the pentasil type, characterised in that borosilicate zeolites, ferrosilicate zeolites and aluminosilicate zeolites of the pentasil type are used, with the exception of the types doped with Tl, Pb or Co, the acidic aluminosilicate zeolites being aftertreated with an acid and/or steam or being doped with transition metals or rare earth metals.

2. Process according to claim 1,
characterised in that borosilicate zeolites doped with transition metals or with rare earth metals or ferrosilicate zeolites doped with transition metals or with rare earth metals are used.

3. Process according to claim 1,
characterised in that acidic borosilicate zeolites aftertreated with an acid and/or steam or acidic ferrosilicate zeolites aftertreated with an acid and/or steam are used.

4. Process according to one or more of claims 1 to 3,
characterised in that the reaction is carried out in a fixed bed.

5. Process according to one or more of claims 1 to 3,
characterised in that the reaction is carried out in a fluidised bed.

## Revendications

1. Procédé de fabrication de pyridines substituées de formule (I), 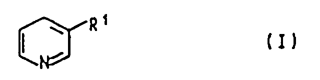 dans laquelle R¹ signifie un alkyle avec 1 à 20 atomes de carbone, un Cycloalkyle-, un radical Aryle- ou Aralkyle par réaction d'un mélange d'acroléine et d'Alcanals de formule (II) 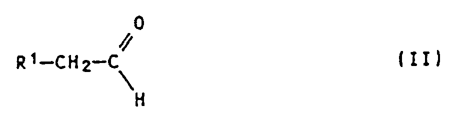 dans laquelle R¹ a la signification donnée ci-dessus, avec l'ammoniac dans la phase gazeuse à des températures allant de 150°C à 400°C en présence de zéolites acides du type Pentasil,
caractérisé en ce qu'
on utilise des zéolites borosilicatées, des zéolites ferrosilicatées et des zéolites à base de silicate d'aluminium du type pentasil à l'exception des types dopés avec Tl, Pb ou Co, dans lequel les zéolites à base de silicate d'aluminium acides sont traités avec un acide et/ou la vapeur d'eau ou sont dopées avec des métaux de transition ou des terres rares.

2. Procédé selon la revendication 1,
caractérisé en ce qu'
on utilise des zéolites borosilicatées ou ferrosilicatées dopées avec des métaux de transition ou des terres rares.

3. Procédé selon la revendication 1,
caractérisé en ce qu'
on utilise des zéolites boro- ou ferrosilicatées retraitées par un acide et/ou par la vapeur d'eau.

4. Procédé selon l'une quelconque ou plusieurs des revendications 1 à 3,
caractérisé en ce qu'
on effectue la réaction en lit fixe.

5. Procédé selon l'une quelconque ou plusieurs des revendications 1 à 3,
caractérisé en ce qu'
on effectue la réaction en lit fluidifié.
